# EUROPEAN PATENT SPECIFICATION

(11) **EP 3 413 825 B1**
(45) Date of publication and mention of the grant of the patent: **26.02.2025**
(21) Application number: 16889737.9
(22) Date of filing: 11.02.2016
(51) Int. Cl.: A61B 18/20, F21V 29/00, H01S 3/04

(54) **COOLING SYSTEM AND METHOD FOR A FLASH BULB OF AN INTENSE PULSED LIGHT DEVICE**
KÜHLSYSTEM UND VERFAHREN FÜR EINE BLITZLAMPE MIT INTENSIV GEPULSTEM LICHT
SYSTÈME ET PROCÉDÉ DE REFROIDISSEMENT POUR UNE AMPOULE DE FLASH D'UN DISPOSITIF DE LUMIÈRE PULSÉE INTENSE

(43) Date of publication of application: 19.12.2018
(73) Proprietor: Leonardo Skin Care Group Ltd, 7040103 Gedera (IL)
(72) Inventor: BAR, Ronny, 7045808 Gedera (IL); MEIR, Ran, 7634940 Rehovot (IL)
(74) Representative: Beck & Rössig European Patent Attorneys
(86) International application number: PCT/IL2016/050167
(87) International publication number: WO 2017/137971

(56) References cited:
- WO-A2-2012/118862
- US-A- 3 638 140
- US-A- 5 481 556
- US-A- 6 017 337
- US-A1- 2003 187 324
- US-A1- 2005 243 539
- US-A1- 2005 243 539
- US-A1- 2007 213 695
- US-A1- 2009 149 930
- US-A1- 2012 061 054
- US-A1- 2015 025 420
- US-B2- 9 027 523

## Description

### FIELD OF THE INVENTION

The present invention relates to skin treatment devices. More particularly, the present invention relates to a cooling system for a flash bulb of an intense pulsed light device. Further, the invention relates to a method for cooling a flash bulb of an intense pulsed light device.

### BACKGROUND OF THE INVENTION

Intense pulsed light, also known as IPL, is a technology used to perform various skin treatments for aesthetic and therapeutic purposes, including hair removal, photo-rejuvenation (for example treatment of skin pigmentation, sun damage and thread veins) and alleviation of dermatologic diseases such as acne. An intense pulsed light device comprises a high- powered, hand-held, computer-controlled flash bulb for delivering intense, visible, broad- spectrum pulses of light, generally in the visible spectral range of 400 to 1,200 nm. Various cut-off filters are commonly used to selectively filter out lower wavelengths, especially potentially damaging ultra violet light. The resulting very high level of light energy is absorbed by the skin at different levels of intensity based on the pigmentation of the tissue. Dark tissues absorb more light than pale tissues. The absorbed light transforms in the tissue to heat. Therefore, dark tissues like pigmented hair follicles or age spots are heated and destroyed, while the surrounding brighter tissues heat less and therefore not damaged.

The generation of the light pulses is based on using a power supply to charge at least one capacitor with high level electrical energy, which is rapidly released in pulses from the at least one capacitor to the flash bulb during treatment of a patient. Thus, the efficiency of an intense pulsed light treatment relies on the energy level of the emitted light, which is expressed in terms of joules per cm², and the pulsing rate, which is expressed in terms of number of pulses per second. A more efficient and rapid treatment is achieved with a high level of emitted light energy and a high pulsing rate.

During operation, intense pulsed light devices generate heat from various sources: the power supply, the at least one capacitor, and the electronics of the system. In addition, the flash bulb generates a significant amount of heat due to the high level of light energy emitted from the flash bulb. The generated heat should be dissipated in order to avoid damage to the system in general and the flash bulb in particular. In the first intense pulsed light devices the heat generated by the flash bulb was dissipated by using cooling systems based on air flow. However, due to the low effectiveness of these cooling systems they were replaced by closed circulating liquid cooling systems, where in general a cooling liquid flows in the vicinity of the flash bulb and absorbs heat from the flash bulb; continues to flow through a cooling member where the heat is dissipated from the cooling liquid; and then flows into a reservoir in which the cooling liquid is stored for further circulation. Currently, the cooling member used in cooling systems of intense pulsed light devices is an air-cooled heat exchanger, for example a radiator, which comprises a fan for dissipating the heat from the cooling liquid that flows through the heat exchanger. Alternatively, the cooling member is a thermoelectric cooler-cooled heat exchanger that absorbs heat from the cooling liquid that flows through the heat exchanger by using a Peltier mechanism. However, the cooling efficiency of the currently available cooling systems for the flash bulb of intense pulsed light devices is limited. When water is used as a cooling liquid, it is heated to substantially 45-60°C when it absorbs the heat of the flash bulb. Cooling the water with a heat exchanger comprising either a fan or a thermoelectric cooler decreases the water temperature to substantially 35-40°C. Such warm water has a low heat absorbance capacity when used for cooling the flash bulb.

Therefore, the currently available cooling systems for the flash bulb of intense pulsed light devices allow only limited operation of the intense pulsed light device. Emission of light in a high energy level and a high pulsing rate produces a great amount of heat that the currently available cooling systems are not able to efficiently dissipate. Therefore, in order to avoid over heating of the flash bulb, emission of light pulses in a high energy level is possible only with a low pulsing rate, or alternatively emission of light pulses in a high pulsing rate is possible only with a low energy level. For example, emission of light in an energy level of up to substantially 40 joules per cm² allows a pulsing rate of only substantially one pulse per minute, whereas emission of light in a pulsing rate of substantially five pulses per second allows emission of light with an energy level of up to only substantially 10 joules per cm². The result in both cases is a treatment that lasts for a long period of time until a desired result is achieved. For example, in a hair removal treatment, it is necessary to repeat the treatment on a certain area of the skin several times until all the hair follicles in this area are destroyed. To summarize, the heat dissipation capacity of the currently available cooling systems is a limiting factor in the operation of intense pulsed light devices.

Therefore, there is a long felt need for a cooling system for intense pulsed light devices that cools the flash bulb down to a level that allows emission of light pulses in a high energy level and a high pulsing rate without causing damage to the intense pulsed light device in general, and the flash bulb in particular. More particularly, there is long felt need for a cooling system for intense pulsed light devices that cools the flash bulb down to a level that allows emission of light pulses in an energy level of up to substantially 40 joules per cm² and a pulsing rate of substantially 5 pulses per second.

A cooling system for a flash bulb of an intense pulsed light device comprising the features of the preamble portion of claim 1 and a method for cooling a flash bulb of an intense pulsed light device comprising the features of the preamble portion of claim 7 are known from US 2005/243539 A1.

Additional cooling elements are known from US 2003/187324 A1.

The present invention aims at more efficient cooling.

### SUMMARY OF THE INVENTION

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control.

In order to solve the above-indicated technical problem, the present invention provides a cooling system for a flash bulb of an intense pulsed light device as defined by claim 1 and a method for cooling a flash bulb of an intense pulsed light device as defined by claim 7.

Advantageous embodiments are indicated in further claims.

### BRIEF DESCRIPTION OF THE DRAWINGS

Embodiments are herein described, by way of example only, with reference to the accompanying drawings. With specific reference now to the drawings in detail, it is stressed that the particulars shown are by way of example and for purposes of illustrative discussion of the preferred embodiments, and are presented in the cause of providing what is believed to be the most useful and readily understood description of the principles and conceptual aspects of the embodiments. In this regard, no attempt is made to show structural details in more detail than is necessary for a fundamental understanding, the description taken with the drawings making apparent to those skilled in the art how several forms may be embodied in practice. In the drawings:
- Fig. 1 schematically illustrates, according to an exemplary embodiment, a diagrammatic presentation of a cooling system for a flash bulb of an intense pulsed light device.
- Fig. 2 schematically illustrates a diagrammatic presentation of additional embodiments of a cooling system for a flash bulb of an intense pulsed light device.

### DESCRIPTION OF THE PREFERRED EMBODIMENTS

Before explaining at least one embodiment in detail, it is to be understood that the invention is not limited in its application to the details of construction and the arrangement of the components set forth in the following description or illustrated in the drawings. The invention is defined by the claims.

Also, it is to be understood that the phraseology and terminology employed herein is for the purpose of description and should not be regarded as limiting. In discussion of the various figures described herein below, like numbers refer to like parts. The drawings are generally not to scale.

For clarity, non-essential elements were omitted from some of the drawings.

One aim of the present invention is to provide a cooling system for a flash bulb of an intense pulsed light device.

Another aim of the present invention is to provide a cooling system for a flash bulb of an intense pulsed light device that allows emission of light pulses in a high energy level and a high pulsing rate.

A further aim of the present invention is to provide a method for cooling a flash bulb of an intense pulsed light device. Yet a further aim of the present invention is to provide a method for cooling a flash bulb of an intense pulsed light device that allows emission of light pulses in a high energy level and a high pulsing rate. According to one aspect, a cooling system for a flash bulb of an intense pulsed light device is provided.

The term "cooling system" as disclosed herein refers to a cooling system for a flash bulb of an intense pulsed light device.

The term "flash bulb" as disclosed herein refers to a flash bulb of an intense pulsed light device.

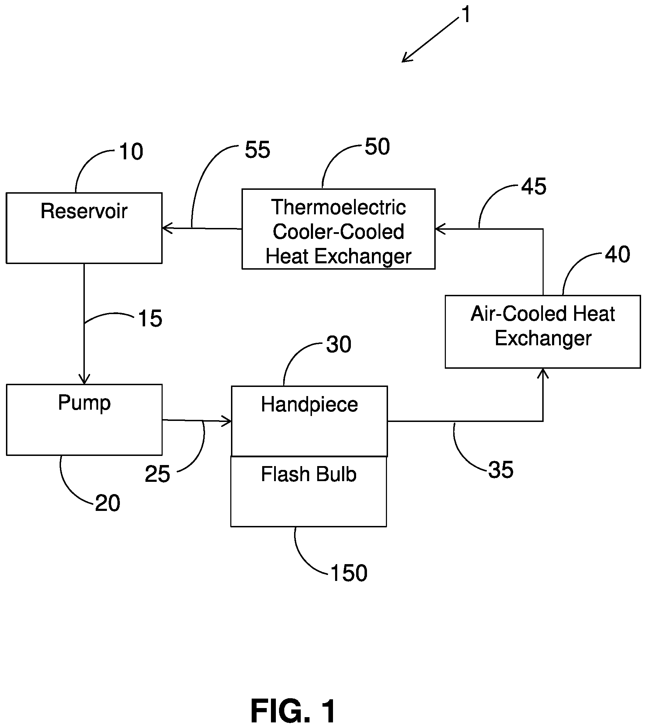

Fig. 1 schematically illustrates, according to an exemplary embodiment, a diagrammatic presentation of a cooling system for a flash bulb of an intense pulsed light device. The cooling system 1 is a closed circulating liquid cooling system. The cooling system 1 comprises a reservoir 10, a pump fluidically connected with a conduit 15 downstream to the reservoir 10, a handpiece 30 thermally connected to a flash bulb 150 and fluidically connected downstream to the pump 20 with a conduit 25, an air-cooled heat exchanger 40 fluidically connected downstream to the handpiece 30 with a conduit 35, and a thermoelectric cooler-cooled heat exchanger 50 fluidically connected downstream to the air-cooled heat exchanger 40 with a conduit 45 and fluidically connected upstream to the reservoir 10 with a conduit 55. A cooling liquid flows in the cooling system 1. According to an embodiment, the cooling liquid is water.

The reservoir 10 is configured to store a cooling liquid, for example water. According to one embodiment, the reservoir 10 is made of a thermally conductive material. According to another embodiment, the reservoir 10 is made of a water-proof material. According to a preferred embodiment, the reservoir 10 is made of a water-proof thermally conductive material, for example stainless steel. Thus, heat carried by the cooling liquid, may be dissipated from the cooling liquid to the ambient air while stored in the reservoir 10. According to still another embodiment, the surface area of the reservoir 10 is large and configured to facilitate a rapid transfer of heat from the cooling liquid stored in the reservoir 10 to the ambient air.

The pump 20 is fluidically connected downstream to the reservoir 10 with a conduit 15 and upstream to the handpiece 30 with a conduit 25. The pump 20 is configured to draw cooling liquid from the reservoir 10 and push the cooling liquid towards the handpiece 30. Furthermore, the pump 20 is configured to drive the flow of the cooling liquid throughout the cooling system 1, namely from the reservoir 10 through the various components of the cooling system 1 and back to the reservoir 10.

The handpiece 30 is configured to hold a flash bulb 150, which is configured to emit pulses of high energy light. According to one embodiment, the handpiece 30 is configured to be held and manipulated by a hand of a user. The handpiece 30 is thermally connected to the flash bulb 150. In other words, the handpiece 30 is configured to allow the flow of a cooling liquid in the vicinity of the flash bulb 150 in a manner that allows transfer of heat from the flash bulb 150 to the cooling liquid. The heat that is generated as a result of the high intensity of light that is emitted by the flash bulb 150, which is transferred to the cooling liquid that flows in the handpiece 30, may cause an increase of the temperature of the cooling liquid to a level of, for example, substantially 45-60°C, depending on the energy level and pulsing rate of the light emitted by the flash bulb 150. The heated cooling liquid then flows from the handpiece 30 to an air-cooled heat exchanger 40 through a conduit 35.

According to some embodiments, the air-cooled heat exchanger 40 is configured to transfer heat from the cooling liquid that flows through the air-cooled heat exchanger 40 to the ambient air. According to a preferred embodiment, the air-cooled heat exchanger 40 is of a radiator-type. According to another embodiment, the air-cooled heat exchanger 40 comprises at least one, preferably two fans, in order to facilitate the transfer of heat from the cooling liquid that flows through the air-cooled heat exchanger 40 to the ambient air. According to one embodiment, the air-cooled heat exchanger 40 is configured to decrease the temperature of the cooling liquid down to substantially 35-40°C. According to another embodiment, the air-cooled heat exchanger 40 is configured to decrease the temperature of the cooling liquid down to ambient temperature. According to a preferred embodiment, the air-cooled heat exchanger 40 is configured to decrease the temperature of the cooling liquid to substantially 28°C. Afterwards, the cooling liquid continues to flow from the air-cooled heat exchanger 40 to the thermoelectric cooler-cooled heat exchanger 50 through a conduit 45.

According to some embodiments, the thermoelectric cooler-cooled heat exchanger 50 is configured to absorb heat from the cooling liquid while consuming an electrical energy, using for example a Peltier effect. According to other embodiments, the thermoelectric cooler-cooled heat exchanger 50 further comprises at least one, preferably two fans, in order to facilitate transfer of heat from the thermoelectric cooler to the ambient air, to further enhance the cooling of the cooling liquid. According to one embodiment, the thermoelectric cooler-cooled heat exchanger 50 is configured to decrease the temperature of the cooling liquid down to less than substantially 28°C. According to another embodiment, the thermoelectric cooler-cooled heat exchanger 50 is configured to decrease the temperature of the cooling liquid down to substantially 26°C. According to yet another embodiment, the thermoelectric cooler-cooled heat exchanger 50 is configured to cool the cooling liquid when the temperature of the cooling liquid is above a predetermined threshold temperature. According to a preferred embodiment, the threshold temperature is substantially 28°C. Thus, only when the temperature of the cooling liquid that enters the thermoelectric cooler-cooled heat exchanger 50 is above the threshold temperature, for example substantially 28°C, the thermoelectric cooler-cooled heat exchanger 50 is configured to be actuated and cool the cooling liquid to a temperature below the threshold temperature. This embodiment allows saving of energy, and elimination of physical deterioration of the thermoelectric cooler, when the cooling liquid that enters the thermoelectric cooler-cooled heat exchanger 50 is cool enough and there is no need to further cool it. After flowing through the thermoelectric cooler-cooled heat exchanger 50, the cooling liquid flows into the reservoir 10 through a conduit 55.

According to the embodiment illustrated in Fig, 1, the air-cooled heat exchanger 40 is located upstream to the thermoelectric cooler-cooled heat exchanger 50. The thermoelectric cooler-cooled heat exchanger 50 may be located upstream to the air-cooled heat exchanger 40. However, the latter configuration is not efficient in terms of energy saving, elimination of physical deterioration of the thermoelectric cooler and achievement of a desired temperature of the cooling liquid. The cooling liquid that exits the handpiece 30 is in a relatively high temperature, for example in the range of substantially 45- 60°C. Thus, a great amount of electrical energy should be consumed by the thermoelectric cooler-cooled heat exchanger 50 in order to cool the cooling liquid to ambient temperature. Furthermore, because of the extensive operation of the thermoelectric cooler-cooled heat exchanger 50, a rapid physical deterioration of the thermoelectric cooler is expected. In addition, the air-cooled heat exchanger 40 is expected to have a low effectiveness in further cooling the cooling liquid that exits the thermoelectric cooler-cooled heat exchanger 50, since air cooling is not sufficient for further lowering the temperature of a cooling liquid that already exists the thermoelectric cooler-cooled heat exchanger 50 in a temperature close to ambient temperature.

Therefore, the configuration in which the air-cooled heat exchanger 40 is located upstream to the thermoelectric cooler-cooled heat exchanger 50, as illustrated in Fig. 1, was found to be efficient in terms of energy saving, elimination of physical deterioration of the thermoelectric cooler and achieving a low temperature of the cooling liquid. This configuration allows firstly the cooling of the cooling liquid that exits the handpiece 30 to a temperature close to ambient temperature by the air-cooled heat exchanger 40 in an economical manner without consuming a high amount of energy. In addition, further cooling of the cooling liquid by the thermoelectric cooler-cooled heat exchanger 50 is more efficient, economical and does not require extensive operation of the thermoelectric cooler.

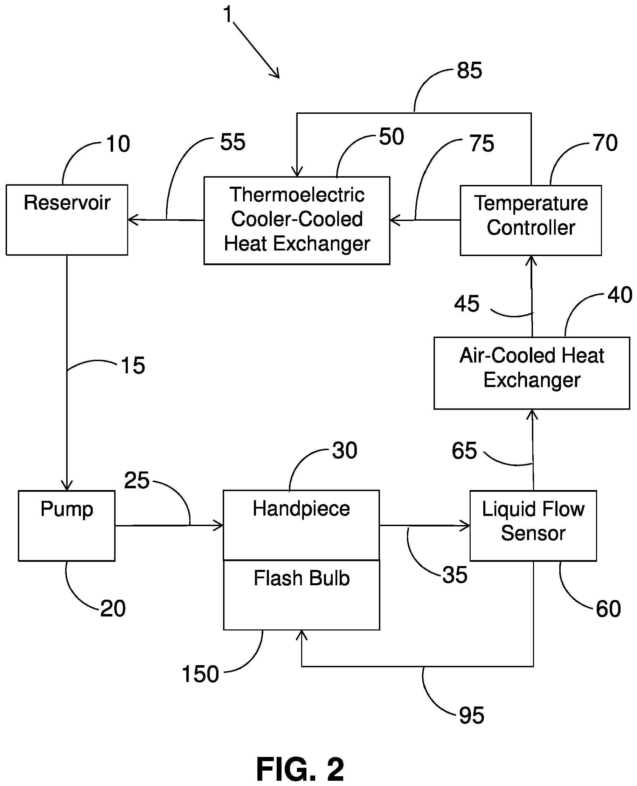

Fig. 2 schematically illustrates a diagrammatic presentation of additional embodiments of a cooling system for a flash bulb of an intense pulsed light device. In addition to the aforementioned components, illustrated in Fig. 1, the cooling system 1 further comprises a liquid flow sensor 60. According to one embodiment, the liquid flow sensor 60 may be installed in any position in the cooling system 1. According to a preferred embodiment, illustrated in Fig. 2, the liquid flow sensor 60 is located downstream to the handpiece 30, and is fluidically connected to the handpiece 30 with a conduit 35, and to the air-cooled heat exchanger 40 with a conduit 65. According to one embodiment, the liquid flow sensor 60 is further electronically connected with a connection line 95 to the flash bulb 150. According to another embodiment, the liquid flow sensor 60 is further electronically connected with a connection line (not shown) to a display unit (not shown) configured to display the flow level of the cooling liquid. According to yet another embodiment, the liquid flow sensor 60 is further electronically connected with a connection line (not shown) to a processor (not shown). According to still another embodiment, the liquid flow sensor 60 is further electronically connected with a connection line (not shown) to a computing member of the intense pulsed light device (not shown). The liquid flow sensor 60 is configured to sense whether there is flow of cooling liquid in the system, and allow for example automatic shutoff of the flash bulb 150 once flow of cooling liquid in not detected, for example due to malfunction of the cooling system 1.

The cooling system 1 further comprises a temperature controller 70. The temperature controller 70 is installed downstream to the air-cooled heat exchanger 40, and is fluidically connected to the air-cooled heat exchanger 40 with a conduit 45, and to the thermoelectric cooler-cooled heat exchanger 50 with a conduit 75. The temperature controller 70 is further electronically connected to the thermoelectric cooler-cooled heat exchanger 50 with a connection line 85. The temperature controller 70 is configured to sense the temperature of the cooling liquid that enters into the thermoelectric cooler-cooled heat exchanger 50. The temperature controller 70 is configured to control the operation of the thermoelectric cooler- cooled heat exchanger 50 according to the temperature of the cooling liquid that enters into the thermoelectric cooler-cooled heat exchanger 50.

One way of controlling the operation of the thermoelectric cooler 50 is by determining a threshold temperature. According to a preferred embodiment, the threshold temperature is substantially 28°C. Thus, according to one embodiment, when the temperature of the cooling liquid that is sensed by the temperature controller 70 is above the predetermined threshold temperature, the temperature controller 70 actuates the thermoelectric cooler-cooled heat exchanger 50 in order to decrease the temperature of the cooling liquid to a temperature below the predetermined threshold temperature. On the other hand, when the temperature of the cooling liquid that is sensed by the temperature controller 70 is below the predetermined threshold temperature, the temperature controller 70 shuts off the thermoelectric cooler-cooled heat exchanger 50, in order to save energy and eliminate physical deterioration of the thermoelectric cooler when the temperature of the cooling liquid is sufficiently low. According to a further embodiment, the temperature controller 70 is electronically connected with a connection line (not shown) to a display unit (not shown) configured to show the temperature of the cooling liquid that enters into the thermoelectric cooler-cooled heat exchanger 50. According to still a further embodiment, the temperature controller 70 is electronically connected with a connection line (not shown) to a processor (not shown). According to yet a further embodiment, the temperature controller 70 is electronically connected with a connection line (not shown) to a computing member of the intense pulsed light device (not shown). According to a one embodiment, all the connection lines disclosed herein are wired.

According to another embodiment, all the connection lines disclosed herein are unwired.

According to one aspect, a method for cooling a flash bulb 150 of an intense pulsed light device is provided.

The term "cooling method" as disclosed herein relates to a method for cooling a flash bulb of an intense pulsed light device.

According to one embodiment, the cooling method comprises:
flowing a cooling liquid by using a pump 20 from a reservoir 10 through a handpiece
30 thermally connected to the flash bulb 150;
decreasing the temperature of the cooling liquid by flowing the cooling liquid through an air-cooled heat exchanger 40;
further decreasing the temperature of the cooling liquid by flowing the cooling liquid through an actuated thermoelectric cooler-cooled heat exchanger 50;
flowing the cooling liquid into the reservoir 10.

According to another embodiment, the decreasing the temperature of the cooling liquid by flowing the cooling liquid through an air-cooled heat exchanger is down to a temperature of substantially 35-40°C

According to yet another embodiment, the decreasing the temperature of the cooling liquid by flowing the cooling liquid through an air-cooled heat exchanger is down to ambient temperature.

According to still another embodiment, the decreasing the temperature of the cooling liquid by flowing the cooling liquid through an air-cooled heat exchanger is down to a temperature of substantially 28°C. According to an additional embodiment, the further decreasing of the temperature of the cooling liquid by flowing the cooling liquid through an actuated thermoelectric cooler- cooled heat exchanger 50 is down to a temperature less than substantially 28°C. According to yet an additional embodiment, the further decreasing of the temperature of the cooling liquid by flowing the cooling liquid through an actuated thermoelectric cooler- cooled heat exchanger 50 is down to a temperature of substantially 26°C.

According to still an additional embodiment, the thermoelectric cooler-cooled heat exchanger 50 is configured to be actuated when the temperature of the cooling liquid that enters the thermoelectric cooler-cooled heat exchanger 50 is above a predetermined threshold temperature.

According to another embodiment, the thermoelectric cooler-cooled heat exchanger 50 is configured to be actuated when the temperature of the cooling liquid that enters the thermoelectric cooler-cooled heat exchanger 50 is above a predetermined threshold temperature of substantially 28°C.

### Examples

An intense pulsed light device comprising a cooling system 1 of the present invention was used for a hair removal treatment. Pulsed light was emitted in an energy level of substantially 40 joules per cm2 and a pulsing rate of substantially five pulses per second. An entire body hair removal treatment, including hair removal from both arms, legs, axilla and groins was completed within 20 minutes. A similar treatment using a pulsed light device comprising a prior art cooling system, which comprises only either an air-cooled heat exchanger, or a thermoelectric cooler-cooled heat exchanger, can be performed with an emitted light in an energy level of up to substantially 40 joules per cm2 and a pulsing rate of substantially one pulse per second, or an emitted light in an energy level of up to substantially 10 joules per cm2 and a pulsing rate of substantially five pulses per second. An entire body hair removal treatment, including hair removal from both arms, legs, axilla and groins, with an intense pulsed light device comprising a prior art cooling system is completed only within 1.5-2.5 hours.

These results demonstrate the advantage of using an intense pulsed light device comprising a cooling system 1 of the present invention over an intense pulsed light device comprising a prior art cooling system, namely a significant decrease in hair removal treatment time duration - from 1.5-2.5 hours down to only 20 minutes. Obviously, such a decrease in treatment time period has further beneficial implications, for example in terms of cost effectiveness and comfort of treatment to the patient and the operator.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub combination.

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims.

## Claims

1. A cooling system for a flash bulb (150) of an intense pulsed light device, the cooling system comprising:
a reservoir (10);
a pump (20) fluidically connected with a conduit downstream to the reservoir (10);
a handpiece (30) thermally connected to a flash bulb of an intense pulsed light device and fluidically connected downstream to the pump with a conduit (25);
an air-cooled heat exchanger (40) fluidically connected downstream to the handpiece with a conduit (35); and
a thermoelectric cooler-cooled heat exchanger (50) fluidically connected downstream to the air-cooled heat exchanger with a conduit (45) and fluidically connected upstream to the reservoir (10) with a conduit (55),
wherein a cooling liquid circulates in the cooling system,
**characterized by**
a temperature controller (70), wherein said temperature controller (70) is installed downstream to the air-cooled heat exchanger (40) and is fluidically connected to the air-cooled heat exchanger (40) with a conduit (45), and to the thermoelectric cooler-cooled heat exchanger (50) with a conduit (75), wherein said temperature controller (70) is electronically connected to the thermoelectric cooler-cooled heat exchanger (50) with a connection line (85), wherein said temperature controller (70) is configured to sense the temperature of the cooling liquid that enters into the thermoelectric cooler-cooled heat exchanger (50), wherein said temperature controller (70) is configured to control operation of the thermoelectric cooler-cooled heat exchanger (50) according to the temperature of the cooling liquid that enters into the thermoelectric cooler-cooled heat exchanger (50) by using a predetermined threshold temperature, and
wherein the thermoelectric cooler-cooled heat exchanger (50) is configured to be actuated by the temperature controller when the sensed temperature of the cooling liquid is above said predetermined threshold temperature.

2. The cooling system of claim 1, wherein the pump (20) is configured to drive the flow of the cooling liquid throughout the cooling system.

3. The cooling system of claim 1 or 2, wherein the air-cooled heat exchanger (40) comprises at least one fan.

4. The cooling system of one of claims 1 to 3, wherein the air-cooled heat exchanger is configured to decrease the temperature of the cooling liquid down to substantially 35-40°C.

5. The cooling system of one of claims 1 or 4, wherein the thermoelectric cooler-cooled heat exchanger (50) comprises at least one fan.

6. The cooling system of one of claims 1 to 5, further comprising a liquid flow sensor (60).

7. A method for cooling a flash bulb (150) of an intense pulsed light device, the cooling method comprising:
flowing a cooling liquid by using a pump (20) from a reservoir (10) through a handpiece (30) thermally connected to the flash bulb;
decreasing the temperature of the cooling liquid by flowing the cooling liquid through an air-cooled heat exchanger (40);
further decreasing the temperature of the cooling liquid by flowing the cooling liquid through an actuated thermoelectric cooler-cooled heat exchanger (50); and
flowing the cooling liquid into the reservoir (10),
**characterized by**
sensing the temperature of the cooling liquid that enters into the thermoelectric cooler-cooled heat exchanger (50) with a temperature controller (70),
controlling, by said temperature controller (70), operation of the thermoelectric cooler-cooled heat exchanger (50) according to the temperature of the cooling liquid that enters into the thermoelectric cooler-cooled heat exchanger (50) by using a predetermined threshold temperature, and
actuating the thermoelectric cooler-cooled heat exchanger (50) by the temperature controller when the sensed temperature of the cooling liquid is above said predetermined threshold temperature.

8. The cooling method of claim 7, wherein the pump (20) is configured to drive the flow of the cooling liquid from the reservoir (10), through the handpiece (30), the air-cooled heat exchanger (40), the thermoelectric-cooler-cooled heat exchanger (50) and back to the reservoir (10).

9. The cooling method of claim 7 or 8, wherein the air-cooled heat exchanger (40) comprises at least one fan.

10. The cooling method of one of claims 7 to 9, wherein the decreasing the temperature of the cooling liquid by flowing the cooling liquid through the air-cooled heat exchanger (40) is down to a temperature of substantially 35-40°C.

11. The cooling method of one of claims 7 to 10, wherein the thermoelectric cooler-cooled heat exchanger (50) comprises at least one fan.

12. The cooling method of one of claims 7 to 11, wherein the decreasing the temperature of the cooling liquid by flowing the cooling liquid through the thermoelectric cooler-cooled heat exchanger (50) is down to a temperature less than substantially 28°C.

## Patentansprüche

1. Kühlsystem für eine Blitzröhre (150) einer Vorrichtung für intensives gepulstes Licht, umfassend:
ein Reservoir (10);
eine Pumpe (20), die mit einer Leitung stromabwärts des Reservoirs (10) in Fluidverbindung steht;
ein Handstück (30), das mit einer Blitzröhre einer Vorrichtung für intensives gepulstes Licht in thermischer Verbindung steht und mit einer Leitung (25) stromabwärts der Pumpe in Fluidverbindung steht;
einen luftgekühlten Wärmetauscher (40), der mit einer Leitung (35) stromabwärts des Handstücks in Fluidverbindung steht; und
einen durch einen thermoelektrischen Kühler gekühlten Wärmetauscher (50), der mit einer Leitung (45) stromabwärts des luftgekühlten Wärmetauschers in Fluidverbindung steht und mit einer Leitung (55) stromaufwärts des Reservoirs (10) in Fluidverbindung steht,
wobei eine Kühlflüssigkeit in dem Kühlsystem zirkuliert,
**gekennzeichnet durch**
eine Temperatursteuer- und -regeleinrichtung (70),
wobei die Temperatursteuer- und -regeleinrichtung (70) stromabwärts des luftgekühlten Wärmetauschers (40) installiert ist und mit dem luftgekühlten Wärmetauscher (40) über eine Leitung (45) und mit dem thermoelektrischen kühlergekühlten Wärmetauscher (50) über eine Leitung (75) in Fluidverbindung steht,
wobei die Temperatursteuer- und -regeleinrichtung (70) mit dem durch den thermoelektrischen Kühler gekühlten Wärmetauscher (50) über eine Verbindungsleitung (85) elektronisch verbunden ist,
wobei die Temperatursteuer- und -regeleinrichtung (70) derart konfiguriert ist, dass sie die Temperatur der Kühlflüssigkeit erfasst, die in den durch den thermoelektrischen Kühler gekühlten Wärmetauscher (50) eintritt,
wobei die Temperatursteuer- und -regeleinrichtung (70) derart konfiguriert ist, dass sie den Betrieb des durch den thermoelektrischen Kühler gekühlten Wärmetauschers (50) gemäß der Temperatur der Kühlflüssigkeit, die in den durch den thermoelektrischen Kühler gekühlten Wärmetauscher (50) eintritt, steuert durch Verwendung einer vorgegebenen Schwellentemperatur, und
wobei der durch den thermoelektrischen Kühler gekühlte Wärmetauscher (50) derart konfiguriert ist, durch die Temperatursteuer- und -regeleinrichtung betätigt zu werden, wenn die erfasste Temperatur der Kühlflüssigkeit über der vorgegebenen Schwellentemperatur liegt.

2. Kühlsystem nach Anspruch 1, wobei die Pumpe (20) derart konfiguriert ist, dass sie den Fluss der Kühlflüssigkeit durch das Kühlsystem antreibt.

3. Kühlsystem nach Anspruch 1 oder 2, wobei der luftgekühlte Wärmetauscher (40) mindestens einen Lüfter umfasst.

4. Kühlsystem nach einem der Ansprüche 1 bis 3, wobei der luftgekühlte Wärmetauscher derart konfiguriert ist, dass er die Temperatur der Kühlflüssigkeit auf im Wesentlichen 35 bis 40°C senkt.

5. Kühlsystem nach einem der Ansprüche 1 oder 4, wobei der durch den thermoelektrischen Kühler gekühlte Wärmetauscher (50) mindestens einen Lüfter umfasst.

6. Kühlsystem nach einem der Ansprüche 1 bis 5, weiterhin umfassend einen Flüssigkeitsströmungssensor (60).

7. Verfahren zum Kühlen einer Blitzröhre (150) einer Vorrichtung für intensives gepulstes Licht, wobei das Kühlverfahren umfasst:
Leiten einer Kühlflüssigkeit unter Verwendung einer Pumpe (20) von einem Reservoir (10) durch ein Handstück (30), das thermisch mit der Blitzröhre verbunden ist;
Verringern der Temperatur der Kühlflüssigkeit durch Leiten der Kühlflüssigkeit
durch einen luftgekühlten Wärmetauscher (40);
weiteres Absenken der Temperatur der Kühlflüssigkeit durch Leiten der Kühlflüssigkeit durch einen betätigten durch einen thermoelektrischen Kühler gekühlten Wärmetauscher (50); und
Leiten der Kühlflüssigkeit in den Vorratsbehälter (10),
**gekennzeichnet durch**
Erfassen der Temperatur der Kühlflüssigkeit, die in den durch den thermoelektrischen Kühler gekühlten Wärmetauscher (50) eintritt, mittels einer die Temperatursteuer- und -regeleinrichtung (70),
Steuern des Betriebs des durch den thermoelektrischen Kühler gekühlten Wärmetauschers (50) mittels der Temperatursteuer- und -regeleinrichtung (70) gemäß der Temperatur der Kühlflüssigkeit, die in den durch den thermoelektrischen Kühler gekühlten Wärmetauscher (50) eintritt, durch Verwendung einer vorgegebenen Schwellentemperatur, und
Betätigen des durch den thermoelektrischen Kühler gekühlten Wärmetauschers (50) durch die Temperatursteuer- und -regeleinrichtung (70), wenn die erfasste Temperatur der Kühlflüssigkeit über der vorgegebenen Schwellentemperatur liegt.

8. Kühlverfahren nach Anspruch 7, wobei die Pumpe (20) derart konfiguriert ist, dass sie den Fluss der Kühlflüssigkeit aus dem Reservoir (10), durch das Handstück (30), den luftgekühlten Wärmetauscher (40), den thermoelektrischen Wärmetauscher (50) und zurück zum Reservoir (10) antreibt.

9. Kühlverfahren nach Anspruch 7 oder 8, wobei der luftgekühlte Wärmetauscher (40) mindestens einen Lüfter umfasst.

10. Kühlverfahren nach einem der Ansprüche 7 bis 9, wobei die Temperatur der Kühlflüssigkeit durch das Strömen der Kühlflüssigkeit durch den luftgekühlten Wärmetauscher (40) auf eine Temperatur von im Wesentlichen 35-40°C gesenkt wird.

11. Kühlverfahren nach einem der Ansprüche 7 bis 10, wobei der durch den thermoelektrischen Kühler gekühlte Wärmetauscher (50) mindestens einen Lüfter umfasst.

12. Kühlverfahren nach einem der Ansprüche 7 bis 11, wobei die Temperatur der Kühlflüssigkeit durch das Strömen der Kühlflüssigkeit durch den durch den thermoelektrischen Kühler gekühlten Wärmetauscher (50) auf eine Temperatur von weniger als im Wesentlichen 28°C gesenkt wird.

## Revendications

1. Système de refroidissement pour une ampoule de flash (150) d'un dispositif à lumière pulsée intense, le système de refroidissement comprenant:
un réservoir (10);
une pompe (20) reliée fluidiquement à un conduit en aval du réservoir (10);
une pièce à main (30) reliée thermiquement à une ampoule de flash d'un dispositif à lumière pulsée intense et reliée fluidiquement en aval de la pompe par un conduit (25);
un échangeur de chaleur refroidi par air (40) relié fluidiquement en aval de la pièce à main par un conduit (35); et
un échangeur de chaleur refroidi par un refroidisseur thermoélectrique (50) relié fluidiquement en aval de l'échangeur de chaleur refroidi par air par un conduit (45) et relié fluidiquement en amont du réservoir (10) par un conduit (55),
dans lequel un liquide de refroidissement circule dans le système de refroidissement,
**caractérisé par**
un contrôleur de température (70), dans lequel ledit contrôleur de température (70) est installé en aval de l'échangeur de chaleur refroidi par air (40) et est relié fluidiquement à l'échangeur de chaleur refroidi par air (40) par un conduit (45), et à l'échangeur de chaleur refroidi par un refroidisseur thermoélectrique (50) par un conduit (75), ledit contrôleur de température (70) est relié électroniquement à l'échangeur de chaleur refroidi par un refroidisseur thermoélectrique (50) par une ligne de connexion (85), dans lequel ledit contrôleur de température (70) est configuré pour détecter la température du liquide de refroidissement qui entre dans l'échangeur de chaleur refroidi par un refroidisseur thermoélectrique (50), dans lequel ledit contrôleur de température (70) est configuré pour contrôler le fonctionnement de l'échangeur de chaleur refroidi par un refroidisseur thermoélectrique (50) en fonction de la température du liquide de refroidissement qui entre dans l'échangeur de chaleur refroidi par un refroidisseur thermoélectrique (50) en utilisant une température seuil
prédéterminée, et
dans lequel l'échangeur de chaleur refroidi par un refroidisseur thermoélectrique (50) est configuré pour être actionné par le contrôleur de température lorsque la température détectée du liquide de refroidissement est supérieure à ladite température seuil prédéterminée.

2. Système de refroidissement selon la revendication 1, dans lequel la pompe (20) est configurée pour entraîner le flux du liquide de refroidissement dans tout le système de refroidissement.

3. Système de refroidissement selon la revendication 1 ou 2, dans lequel l'échangeur de chaleur refroidi par air (40) comprend au moins un ventilateur.

4. Système de refroidissement selon l'une des revendications 1 à 3, dans lequel l'échangeur de chaleur refroidi par air est configuré pour diminuer la température du liquide de refroidissement jusqu'à substantiellement 35 à 40°C.

5. Système de refroidissement selon l'une des revendications 1 ou 4, dans lequel l'échangeur de chaleur refroidi par un refroidisseur thermoélectrique (50) comprend au moins un ventilateur.

6. Système de refroidissement selon l'une des revendications 1 à 5, comprenant en outre un détecteur de flux de liquide (60).

7. Procédé de refroidissement d'une ampoule de flash (150) d'un dispositif à lumière pulsée intense, le procédé de refroidissement comprenant:
faire circuler un liquide de refroidissement à l'aide d'une pompe (20) depuis un réservoir (10) à travers une pièce à main (30) reliée thermiquement à l'ampoule de flash;
diminuer la température du liquide de refroidissement en faisant circuler le liquide de refroidissement à travers un échangeur de chaleur refroidi par air (40);
diminuer encore davantage la température du liquide de refroidissement en faisant circuler le liquide de refroidissement à travers un échangeur de chaleur refroidi par un refroidisseur thermoélectrique actionné (50); et
faire couler le liquide de refroidissement dans le réservoir (10),
**caractérisé par**
la détection de la température du liquide de refroidissement qui entre dans l'échangeur de chaleur refroidi par un refroidisseur thermoélectrique (50) à l'aide d'un contrôleur de température (70),
le contrôle, par ledit contrôleur de température (70), du fonctionnement de l'échangeur de chaleur refroidi par un refroidisseur thermoélectrique (50) en fonction de la température du liquide de refroidissement qui entre dans l'échangeur de chaleur refroidi par un refroidisseur thermoélectrique (50) en utilisant une température seuil prédéterminée, et
l'actionnement de l'échangeur de chaleur refroidi par un refroidisseur thermoélectrique (50) par le contrôleur de température lorsque la température détectée du liquide de refroidissement est supérieure à ladite température seuil prédéterminée.

8. Procédé de refroidissement selon la revendication 7, dans lequel la pompe (20) est configurée pour entraîner le flux du liquide de refroidissement depuis le réservoir (10), à travers la pièce à main (30), l'échangeur de chaleur refroidi par air (40), l'échangeur de chaleur refroidi par un refroidisseur thermoélectrique (50) et le faire revenir dans le réservoir (10).

9. Procédé de refroidissement selon la revendication 7 ou 8, dans lequel l'échangeur de chaleur refroidi par air (40) comprend au moins un ventilateur.

10. Procédé de refroidissement selon l'une des revendications 7 à 9, dans lequel la diminution de la température du liquide de refroidissement par écoulement du liquide de refroidissement à travers l'échangeur de chaleur refroidi par air (40) est réduite à une température sensiblement comprise entre 35 et 40°C.

11. Procédé de refroidissement selon l'une des revendications 7 à 10, dans lequel l'échangeur de chaleur refroidi par un refroidisseur thermoélectrique (50) comprend au moins un ventilateur.

12. Procédé de refroidissement selon l'une des revendications 7 à 11, dans lequel la diminution de la température du liquide de refroidissement par écoulement du liquide de refroidissement à travers l'échangeur de chaleur refroidi par un refroidisseur thermoélectrique (50) est réduite à une température sensiblement inférieure à 28°C.
